Europäisches Patentamt

European Patent Office

Office européen des brevets

(19)

(11) Veröffentlichungsnummer : **0 072 912**
**B1**

(12) **EUROPÄISCHE PATENTSCHRIFT**

(45) Veröffentlichungstag der Patentschrift :
**11.12.85**

(21) Anmeldenummer : **82106325.2**

(22) Anmeldetag : **15.07.82**

(51) Int. Cl.⁴ : **C 07 C 47/127, C 07 C 29/38,
B 01 J 23/50, B 01 J 23/72,
B 01 J 27/16**

(54) Verfahren zur Herstellung von Glyoxal.

(30) Priorität : **04.08.81 DE 3130800**

(43) Veröffentlichungstag der Anmeldung :
**02.03.83 Patentblatt 83/09**

(45) Bekanntmachung des Hinweises auf die Patenterteilung : **11.12.85 Patentblatt 85/50**

(84) Benannte Vertragsstaäten :
**BE CH DE FR GB IT LI NL**

(56) Entgegenhaltungen :
**EP-A- 0 003 343
EP-A- 0 007 570
EP-A- 0 008 013
DE-A- 2 634 439
DE-A- 2 831 229
US-A- 4 258 216**

(73) Patentinhaber : **BASF Aktiengesellschaft
Carl-Bosch-Strasse 38
D-6700 Ludwigshafen (DE)**

(72) Erfinder : **Sauer, Wolfgang, Dr.
Langstrasse 23
D-6719 Kirchheimbolanden (DE)**
Erfinder : **Halbritter, Klaus, Dr.
Schwarzwaldstrasse 19
D-6800 Mannheim (DE)**
Erfinder : **Engelbach, Heinz, Dr.
Kropsburgstrasse 24
D-6703 Limburgerhof (DE)**

Jouve, 18, rue St-Denis, 75001 Paris, France

## Beschreibung

Die Erfindung betrifft ein neues Verfahren zur Herstellung von Glyoxal durch Oxidation von Glykol in Gegenwart eines Kupfer/Silber-Schichtenkatalysators.

Aus der DE-PS 1 923 048 ist bekannt, daß man Glyoxal durch Dampfphasenoxidation von Ethylenglykol an einem Oxidationskatalysator herstellen kann, der Kupfer in Kombination mit Zinn, Phosphor, Arsen, Antimon oder Wismut enthält. Der Katalysator, der zusätzlich auch Silber enthalten kann, wird als Legierung eingesetzt, vorzugsweise in Form von Drehspänen oder Gaze. Für die Kombination Silber/Kupfer/Phosphor, werden Ausbeuten — bezogen auf umgesetztes Ethylenglykol — zwischen 55 % und 72 % angegeben. Die höheren Werte können allerdings nur unter Inkaufnahme eines unvollständigen Glykolumsatzes erzielt werden. Dies ist in wirtschaftlicher Sicht unbefriedigend, da eine Entfernung des restlichen Glykols aus dem Produkt nur mit großem Aufwand möglich ist. Außerdem werden nach diesem bekannten Verfahren unzureichende Raum-Zeit-Ausbeuten erhalten. Nachteilig ist ferner die umständliche Katalysatorherstellung.

In der DE-PS 1 967 147 wird für die Herstellung von Glyoxal aus Ethylenglykol ein Silberkatalysator vorgeschlagen, der Phosphor enthält. Auch bei diesem Verfahren muß eine Glyoxalausbeute bis 70 %, bezogen auf umgesetztes Ethylenglykol, mit unbefriedigenden Umsätzen erkauft werden. Die Raum-Zeit-Ausbeuten sind ebenfalls unbefriedigend.

Es hat nicht an Versuchen gefehlt, die geschilderten Nachteile zu beseitigen. So wird in der DE-OS 2 634 439 der Vorschlag gemacht, bei der Verwendung der phosphorhaltigen Katalysatoren aus Kupfer und/oder Silber dem Reaktionsgemisch eine Bromverbindung zuzugeben. Dadurch kann zwar die Glyoxalausbeute bei nahezu vollständigem Umsatz auf 80,5 % gesteigert werden. Die Raum-Zeit Ausbeuten lassen jedoch auch bei diesem Verfahren zu wünschen übrig. Außerdem ergeben sich durch die aggressiven Bromverbindungen, die bei Reaktionstemperaturen um 450 °C und in Gegenwart von Sauerstoff zugegeben werden, ernste Korrosionsprobleme. Ferner ist von Nachteil, daß die Kupfer enthaltenden Katalysatoren schnell altern, so daß die Ausbeute abfällt.

Nach den Angaben der DE-OS 2 158 343 sinkt die Selektivität von in der DE-PS 1 923 048 beschriebenen Katalysatoren bei einer 700-stündigen Betriebsdauer von einem Anfangswert von 60 bis 64 % auf 55 % ab. Es ist ferner angegeben, daß man einen Kupfer, Silber und Phosphor enthaltenden Katalysator nach einer 700-stündigen Laufzeit durch eine 12-stündige Reduktion bei 450 °C regenerieren kann. In der DE-OS 2 158 344 wird die Regenerierung eines Kupfer und Phosphor enthaltenden Katalysators beschrieben, bei der über den Katalysator mindestens 1 Tag Sauerstoffüberschuß geleitet wird. Diese Regenerierverfahren haben den Nachteil, daß sie mit einem Produktionsausfall verbunden sind und aufwendige Sicherheitsmaßnahmen erfordern, um die Vermischung der zum Regenerieren verwendeten Luft mit dem Reaktionsgas auszuschließen.

Nach dem Verfahren der DE-OS 2 832 405 wird die Lebensdauer von Kupfer-Katalysatoren erheblich verlängert, wenn man die Dampfphasenoxidation von Ethylenglykol bei Temperaturen von 225 bis 500 °C und in Gegenwart einer unter den Reaktionsbedingungen flüchtigen Phosphorverbindung, wie Trimethylphosphat, durchführt, wobei die Phosphormenge (berechnet P), bezogen auf die Gewichtsmenge an Ethylenglykol, 1 bis 100 ppm beträgt. Die Katalysatoren werden hierbei in Form von Drehspänen, Drahtgeweben, Gazen oder auch als Trägerkatalysatoren eingesetzt. Durch dieses Verfahren wird zwar eine erhebliche Verringerung des Aktivitätsverlustes des Katalysators erzielt, eine dauerhafte Stabilisierung der Katalysatoraktivität auf hohem Niveau aber nicht erreicht.

In der DE-A 2 831 229 wird ein Verfahren zur Herstellung von Diketonen durch Oxidation aliphatischer Diole, wie 1,2-Propandiol, 2,3-Butandiol oder n-Hexandiol beschrieben, bei denen man als Katalysator einen Schichtenkatalysator mit mehreren Schichten Silber- und/oder Kupferkristallen verwendet. Besonders vorteilhafte Ergebnisse werden erhalten, wenn man Gemische aus Silber und Kupfer oder allein Silberkristalle verwendet. Wegen der ungewöhnlichen Reaktivität des Glyoxals, durch die es sich von den in der DE-A 2 831 229 genannten aliphatischen Dicarbonylverbindungen erheblich unterscheidet, konnte der Fachmann von den in der DE-A 2 831 229 beschriebenen Verfahrensmaßnahmen keine entsprechenden Ergebnisse bei der Oxidation von Ethylenglykol zum Zwecke der Glyoxalherstellung erwarten.

In der DE-OS 2 803 318 wird ein Verfahren zur Herstellung von Glyoxal aus Glykol beschrieben, bei dem man die Oxidation an Silberkristallen der Korngröße 0,1 bis 2,5 mm bei einer Verweilzeit von höchstens 0,05 Sekunden und einer Temperatur von 447 bis 707 °C vornimmt. Mit diesem Verfahren erzielt man zwar ein Vielfaches der Raum-Zeit-Ausbeuten, die mit den oben beschriebenen Verfahren erhalten werden, von Nachteil ist jedoch die verhältnismäßig geringe Ausbeute von nur 55 %. Man kann die Ausbeute zwar durch einen Zusatz von Bromoform zum Ausgangsgemisch verbessern, muß dann aber Korrosionsprobleme und Schwierigkeiten hinsichtlich der Produktreinheit in Kauf nehmen.

Eine Verbesserung der Ausbeute läßt sich demgegenüber erzielen, wenn man die heißen Reaktionsgase nach dem Verfahren der DE-OS 2 922 599 mit Wasser oder einer wäßrigen Glyoxallösung kondensiert. Bei einem Umsatz von ca. 98 % wird durch diese Behandlung der Reaktionsgase die Glyoxalausbeute, bezogen auf eingesetztes Glykol, ohne Zusatz von Bromverbindungen von 55 % auf bis zu 62 % gesteigert. Darüberhinaus kann nach diesem Verfahren Glyoxal direkt in der handelsüblichen Form einer 40 %igen wäßrigen Lösung hergestellt werden. Außerdem wird im Vergleich zum Verfahren der DE-OS 2 803 318 die Farbzahl des Glyoxals deutlich verbessert und die Betriebssicherheit durch Vermeidung von Verstopfungen im Kondensationsteil stark erhöht. Nachteilig bei diesem Verfahren ist jedoch die Verunreinigung der hergestellten wäßrigen Glyoxallösung mit Glykolaldehyd, zu dessen Entfernung keine wirtschaftlich vertretbaren Methoden zur Verfügung stehen.

Es ergibt sich somit, daß die bekannten Verfahren zur Herstellung von Glyoxal durch katalytische Oxidation von Ethylenglykol im Hinblick auf einen wünschenswerten unkomplizierten und wirtschaftlichen Betrieb, eine einfache Katalysatorherstellung, lange Standzeit des Katalysators, gute Ausbeute bzw. Raum-Zeit-Ausbeute und Reinheit des hergestellten Glyoxals, nicht befriedigen.

Es wurde nun gefunden, daß man bei der kontinuierlichen Herstellung von Glyoxal durch Oxidation von Ethylenglykol, bei der man das Ethylenglykol mit Sauerstoff und einem Inertgas bei Temperaturen von 450 bis 800 °C über einen Katalysator aus Kupfer und Silber leitet, besonders vorteilhafte Ergebnisse erzielt, wenn der Katalysator aus einer oder mehreren Schichten von Kupferkristallen und einer oder mehreren Schichten von Silberkristallen besteht, wobei die Kupfer- und Silberkristalle eine Korngröße von 0,1 bis 2,5 Millimeter aufweisen und wobei der Anteil an Silberkristallen 55 bis 90 Gew.%, jeweils bezogen auf das Gesamtgewicht des Katalysators, beträgt.

Nach dem neuen Verfahren wird die Oxidation des Glykols mit Sauerstoff in Gegenwart eines Inertgases, wie Stickstoff, z. B. in einem Verhältnis von mindestens 4,4 Mol Inertgas je Mol Sauerstoff, durchgeführt. Das Molverhältnis von Sauerstoff zu Ethylenglykol beträgt zweckmäßig mindestens 0,7 zu 1 und höchstens 1,4 zu 1. Die mit 450 bis 800 °C angegebenen Reaktionstemperaturen sind die im Katalysatorbett auftretenden Temperaturen. Die Verweilzeit des Reaktionsgemisches in der Katalysatorschicht sollte 0,05 Sekunden nicht überschreiten.

Bei dem erfindungsgemäßen Verfahren wird die Oxidation an einem Mehrschichtenkatalysator durchgeführt, der mindestens eine Schicht aus Kupferkristallen und mindestens eine Schicht aus Silberkristallen enthält. Die Gesamtschichtdikke des Katalysators beträgt 5 bis 100, vorzugsweise 20 bis 40 mm. Die Katalysatorschichten sind in dem üblicherweise vertikal aufgestellten Reaktor angeordnet. Das Ausgangsgemisch aus Dampf des Ausgangsstoffes Ethylenglykol und Sauerstoff bzw. Luft wird im allgemeinen von oben nach unten geführt, so daß die oberste Schicht gleichzeitig der dem Ausgangsgemisch zugewandte Teil des Katalysators bedeutet. Bei Reaktoren anderer Bauart, z. B. horizontal angeordneten Reaktoren, oder bei anderer Führung des Ausgangsgemisches gelten alle Angaben der Beschreibung über oberen und unteren Teil des Katalysators sinngemäß.

Bezogen auf das Gesamtgewicht aller Katalysatorteilchen beträgt der Anteil an Kupferkristallen 10 bis 45 Gew.%, insbesondere 20 bis 30 Gew.% und der Anteil an Silberkristallen 55 bis 90 Gew.%, insbesondere 70 bis 80 Gew.%. In einem besonders bevorzugten Fall beträgt der Anteil an Kupferkristallen 25 Gew.%, der an Silberkristallen 75 Gew.% des Gesamtgewichts des Katalysators.

Die Zahl der Kuperkristallschichten kann, bezogen auf die Zahl der Silberkristallschichten, größer, gleich oder kleiner sein. Beispielsweise besteht der Mehrschichtenkatalysator aus ein bis vier, vorzugsweise zwei bis drei Schichten aus Kupferkristallen sowie aus ein bis vier, vorzugsweise zwei bis drei Schichten aus Silberkristallen. Die Kupfer- und Silberkristalle haben eine Korngröße von 0,1 bis 2,5 mm, wobei für die einzelnen Schichten Korngrößenfraktionen von zum Beispiel 0,2 bis 0,4 mm, 0,4 bis 0,75 mm, 0,75 bis 1,0 mm, 1,0 bis 2,5 mm verwendet werden.

Die Korngrößen zweier benachbarter Schichten können in Strömungsrichtung zunehmend, gleichbleibend, z. B. beim Wechsel von einer Kupferschicht zu einer Silberschicht oder umgekehrt, oder abnehmend sein.

Die Kupferkristallschichten können einzeln oder mehrere zusammen oberhalb und/oder unterhalb jeder Silberkristallschicht angeordnet sein. Vorzugsweise verwendet man nur zwei oder drei Kupferschichten und ordnet sie oberhalb und/oder unterhalb der oberen Silberschicht an.

Die Schichtung jeder einzelnen Silber- oder Kupferschicht ist meist regelmäßig, so daß die Schichtdicke der Einzelschicht über den ganzen Schichtquerschnitt hinweg gleich ist. In diesen Fällen hängt die Schichtdicke direkt von den Gewichtsanteilen Gesamtkatalysator und der jeweiligen Korngröße der Teilchen ab. Man kann aber auch eine unregelmäßige Schichtung aller oder mehrerer Schichten oder zweckmäßig einer Silber- oder Kupferschicht vornehmen. Das geschieht z. B. dadurch, daß man in der Mitte, auf den Seiten oder vorteilhaft am Rande der Schicht die Hauptmenge der Katalysatorteilchen aufgibt und entsprechend nur eine kleinere Restmenge auf die übrige Schicht verteilt. Die Schichtdicken zweier benachbarter Schichten können in Strömungsrichtung gesehen zunehmend, gleichbleibend oder abnehmend sein.

Es wurde weiterhin gefunden, daß man die bei der Reaktion als unerwünschte Nebenreaktion ablaufende Bildung von Kohlendioxid vermindern und die Glyoxalausbeute steigern kann, wenn man die Oxidation an dem Kupfer-Silber-Schichtenkatalysator in Gegenwart einer unter den Reaktionsbedingungen flüchtigen Phosphorverbindung durchführt. Da der Zusatz der Phosphorverbindung dabei so bemessen sein muß, daß er die Bildung von Kohlendioxid zwar zurückdrängt, aber nicht zur Desaktivierung des Katalysators im Hinblick auf die Glyoxalbildung aus Glykol führt, setzt man beim erfindungsgemäßen Verfahren soviel der flüchtigen Phosphorverbindung zu, daß die Phosphormenge (berechnet P) 0,5 bis 20 ppm, bezogen auf die Gewichtsmenge an Ethylenglykol, beträgt.

Als unter den Reaktionsbedingungen flüchtige Phosphorverbindungen kommen z. B. Trimethylphosphat, Triethylphosphat, Tri-iso-propylphosphat, Tri-n-propylphosphat, Trimethylphosphit, Triethylphos-

# 0 072 912

phit, Triethylphosphinoxid, Methylphosphonsäurediethylester, Methylphosphonsäuredimethylester oder Ethylphosphonsäurediethylester in Betracht.

Die bei der katalytischen Oxidation erhältlichen dampfförmigen Reaktionsgemische werden auf an sich bekannte Weise aufgearbeitet. Beispielsweise werden sie unmittelbar nach dem Verlassen der Katalysatorschicht mit einer Flüssigkeit in Berührung gebracht, die die unverzügliche Kondensation der Oxidationsprodukte bewirkt. Als Kondensationsflüssigkeit verwendet man Wasser, das kondensierte flüssige Reaktionsgemisch oder ein Gemisch dieser beiden Flüssigkeiten. Besonders vorteilhaft ist es, das kondensierte Reaktionsgemisch als Kondensationsflüssigkeit zu verwenden. Bei der Verwendung von kondensierten Reaktionsgemischen als Kondensationsflüssigkeit wird der überschüssige Teil an Kondensat, welcher nicht als gekühlte Kondensationsflüssigkeit im Kreis geführt wird, als Rohprodukt entnommen und gegebenenfalls einer Aufarbeitung oder weiteren Umsetzung zugeführt.

Das dampfförmige Reaktionsgemisch, das eine Temperatur von 320 bis 650 °C aufweist, bringt man zweckmäßig innerhalb von einer Sekunde mit der Kondensationsflüssigkeit in Berührung. Die Temperatur der Kondensationsflüssigkeit beträgt dabei z. B. − 20 bis 80 °C. Man führt diese Behandlung z. B. so durch, daß man den Strom der heißen Reaktionsgase direkt in einen Quenchraum leitet, der sich in Strömungsrichtung unmittelbar an die Katalysatorschicht anschließt. Man verfährt dabei vorzugsweise so, daß man die Kondensationsflüssigkeit in Form von Tröpfchen mit einem durchschnittlichen Durchmesser von 1 bis 2 000 Mikrometern mit den Reaktionsgasen in Berührung bringt. Die Tröpfchen werden mit Hilfe von an sich üblichen Zerteilungsvorrichtungen, insbesondere von Düsen, erzeugt. Das Einsprühen der Kondensationsflüssigkeit in den Quenchraum nimmt man vorteilhaft so vor, daß die Hauptmenge der Tröpfchen auf den Strom der Reaktionsgase mit einem Winkel von 2 bis 85 °C zur Stromachse auftrifft.

Die Menge an Kondensationsflüssigkeit (in den Beispielen auch als Quenchflüssigkeit bezeichnet) beträgt zweckmäßigerweise 20 bis 100 Gewichtsteile, bezogen auf 1 Gewichtsteil der Reaktionsgase.

Das erfindungsgemäße Verfahren liefert im Vergleich zu den bekannten Verfahren auf einfacherem und wirtschaftlicherem Wege ein besseres Gesamtergebnis in Bezug auf Ausbeute, Raum-Zeit-Ausbeute, Reinheit des Endstoffs und Lebensdauer des Katalysators. Die Katalysatorherstellung bzw. Katalysatoraufarbeitung sind besonders einfach. So können Kupfer- und Silberkristalle der betreffenden Teilchengröße, wie sie auch bei der elektrolytischen Herstellung anfallen, direkt verwendet werden.

Bei dem erfindungsgemäßen Verfahren kann man den Kupfer-Silber-Schichtenkatalysator, der sich durch eine hohe Lebensdauer auszeichnet, in den Fällen, in denen dennoch ein Austausch notwendig sein sollte, leicht ersetzen. So können die Kupfer- und Silber-Schichten mechanisch voneinander getrennt werden, was z. B. durch Einlegen von Kupfer-, Silber- oder Edelstahlnetzen zwischen die Kupfer- und Silberschichten mühelos gestaltet werden kann. Anschließend können sowohl das Kupfer als auch das Silber nahezu verlustfrei elektrolytisch direkt wieder in die katalytisch wirksame Form gebracht werden.

Das nach dem erfindungsgemäßen Verfahren erhaltene Glyoxal, das direkt in der handelsüblichen Form einer 40 %igen wäßrigen Lösung erhalten werden kann, zeichnet sich durch eine vergleichsweise hohe Produktqualität aus, die auch bei langem Betriebszeitraum unverändert bleibt. So liegt der Restgehalt der 40 %igen Glyoxallösung an unumgesetztem Glykol unter 0,1 %, der Gehalt an Glykolaldehyd und Formaldehyd liegt jeweils nur bei ca. 1 %. Da außerdem auch die Farbzahl und die Säurezahl der Glyoxallösungen den in diesem Zusammenhang hohen Anforderungen bei Anwendungen in der Textil-, Papier- und Lederindustrie genügen, ist bei dem erfindungsgemäßen Verfahren keine besondere Reinigungsstufe, z. B. mit Ionenaustauscher, notwendig. Der Zusatz von Halogenverbindungen als Inhibitor, der mit einer Gefahr von Korrosionserscheinungen verbunden ist, wird bei dem erfindungsgemäßen Verfahren vermieden.

Besonders hervorzuheben bei den erfindungsgemäßen Verfahren ist die außerordentlich gute Raum-Zeit-Ausbeute, die im Vergleich mit den bekannten Glyoxalverfahren das geringste Reaktorvolumen ermöglicht. Durch diesen Vorteil können die Investitionskosten erniedrigt werden. Zusammen mit der hohen Glyoxalausbeute gibt dies dem erfindungsgemäßen Verfahren eine sehr gute Wirtschaftlichkeit.

Diese vorteilhaften Ergebnisse sind überraschend. So konnte nicht erwartet werden, daß die einfache schichtenartige Kombination von reinen Kupfer- und Silberkristallen besonderer Korngröße im Vergleich zur bekannten Verwendung von Silber/Kupfer-Legierungen mit weiteren Zusätzen (Promotoren) eine so wesentliche Steigerung der Reaktionsgeschwindigkeit und damit der Raum-Zeit-Ausbeute ermöglicht. Ebenso überraschend ist, daß im Vergleich zu der Verwendung von reinen Silberkristallen nicht nur die Ausbeute deutlich gesteigert, sondern auch gleichzeitig der Nebenproduktspiegel drastisch gesenkt wird. Auch die ausbeutesteigernde Wirkung von flüchtigen Phosphorverbindungen ist überraschend, da in der DE-OS 2 832 405 eine positive Wirkung von flüchtigen Phosphorverbindungen nur im Hinblick auf die Verlängerung der Lebensdauer von Kupferkatalysatoren erwähnt wird.

Bei den Kupfer-Silber-Schichtenkatalysatoren, die beim erfindungsgemäßen Verfahren verwendet werden, treten während des Dauerbetriebes keine merkbaren Aktivitätsverluste auf, so daß Maßnahmen zum Zwecke der Verlängerung der Lebensdauer der Katalysatoren überflüssig sind. Es ist daher überraschend, daß der Zusatz von flüchtigen Phosphorverbindungen die Ausbeute durch Verringerung der Kohlendioxidbildung um einen bestimmten Betrag erhöht.

Die in den folgenden Beispielen angeführten Teile bedeuten Gewichtsteile.

4

**0 072 912**

## Beispiel 1

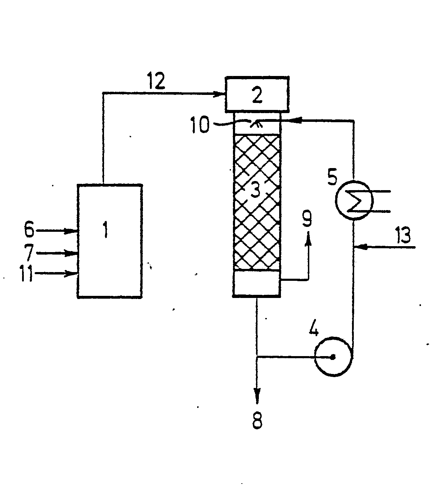

(die Ziffern in Klammern beziehen sich auf die Figur)

Man verwendet eine Anlage mit Ethylenglykol-Verdampfer (1), entsprechenden Zuführungen von Ethylenglykol (6), Wasser (7) und Luft bzw. Stickstoff (11) und einem senkrechten Rohrreaktor (2). Der Reaktor enthält an seinem Kopf die Zuführung (12) für das dampfförmige Ausgangsgemisch und die Reaktorhaube. Die Katalysatorschicht liegt unterhalb des Reaktorkopfes, weiter unten folgt der Quenchraum (10), der den Kopf einer Füllkörperkolonne (3) bildet. Das Kondensat wird teilweise als Quenchflüssigkeit verwendet. Die Quenchflüssigkeit wird mit einer Pumpe (4) über einen Wärmetauscher (5) und ein Düsensystem unmittelbar hinter der Katalysatorschicht in die heißen Gase gesprüht, die im Quenchraum abgekühlt und teilweise kondensiert werden, so daß über die Leitung (8) die Glyoxallösung abgenommen werden kann. Das Abgas entweicht über die Leitung (9).

In den Reaktor (2) wird ein Katalysator aus Kupfer- und Silberkristallen (41 Teile) folgender Zusammensetzung eingetragen (Reihenfolge der Schichten von oben nach unten in Strömungsrichtung der Gase) :

|  | Material | Korngröße (mm) | Anteil am Katalysator (Gew.%) |
|---|---|---|---|
| Schicht 1 | Kupfer | 0,2 - 0,4 | 6,8 |
| Schicht 2 | Kupfer | 0,4 - 0,75 | 18,6 |
| Schicht 3 | Silber | 0,4 - 0,75 | 54,7 |
| Schicht 4 | Silber | 1,0 - 2,5 | 19,9 |

Die Höhe der gesamten Katalysatorschicht beträgt 30 mm. Dem Verdampfer (1) wird pro Stunde ein Gemisch von 220 Teilen Ethylenglykol, 220 Teilen Wasser, 536 Teilen Luft und 1 576 Teilen Stickstoff zugeführt, wo es erhitzt und verdampft wird. Das dampfförmige Ausgangsgemisch wird durch den Katalysator geleitet und bei 600 °C und 1,2 bar umgesetzt. Die Verweilzeit im Katalysatorraum beträgt 0,006 Sekunden.

Die Strömungsgeschwindigkeit der Reaktionsgase beim Verlassen der Katalysatorschicht beträgt 4,8 Meter pro Sekunde. Die Temperatur der Quenchflüssigkeit beträgt 61 °C. Als Quenchflüssigkeit wird zu Beginn Wasser vorgelegt. Entsprechend der Menge an anfallender kondensierter Reaktionsmischung wird ein Teil der Quenchflüssigkeit kontinuierlich über die Leitung 8 abgezogen. Nach einer Einfahrphase erreicht die Quenchflüssigkeit durch Aufkonzentration mit kondensierter Reaktionsmischung eine Stationärkonzentration, die deren Zusammensetzung entspricht. Danach wird mit der Bilanzierung der aus dem Quenchkreislauf entsprechend der Menge an kondensierender Reaktionsmischung kontinuierlich entnommenen Produktlösung begonnen. Zum Verdüsen der Quenchflüssigkeit werden 2 Ringe mit jeweils 6 auf Lücke angeordneten Düsen verwendet. Die Düsen eines Ringes befinden sich an der Kolonnenwand und sind symmetrisch angeordnet. Der Auftreffwinkel der Tröpfchen zur Stromachse aller Düsen ist unterschiedlich und beträgt 15 bis 75 °C. Bei 70 % der Tröpfchen beträgt dieser Winkel 30 bis 75°. Die Tröpfchen haben einen durchschnittlichen Durchmesser von 200 Mikrometern. Man erhält 363 Teile pro Stunde einer 40-gewichtsprozentigen Glyoxallösung, das sind 145 Teile Glyoxal pro Stunde, entsprechend einer Ausbeute von 70,6 % der Theorie, bezogen auf verwendetes Glyoxal. Die Lebensdauer des Katalysators beträgt 90 Tage. Die Glyoxallösung hat einen Gehalt von 0,05 Gewichtsprozent Ethylenglykol, 1,2 Gewichtsprozent Formaldehyd, 0,9 Gewichtsprozent Glykolaldehyd und eine Säurezahl von 4 (Kaliumhydroxidmenge in Milligramm, die zur Neutralisation von einem Gramm Glyoxallösung benötigt wird).

Der Umsatz beträgt 99,9 Prozent, die Raum-Zeit-Ausbeute 15,4 g Glyoxal pro $cm^3$ Katalysatorvolumen und Stunde. Farbzahl der Lösung nach dreitägigem Betrieb : 12 (die Farbzahl wird mittels der Platin-Cobalt-Skala nach ASTM D 1209-69 bestimmt).

## Beispiel 2

(Vergleichsbeispiel, bei dem der Schichtenkatalysator nur aus Silber besteht)

Es wird die gleiche Anlage wie in Beispiel 1 verwendet. In den Reaktor wird ein Schichtenkatalysator wie in Beispiel 1 eingetragen, wobei jedoch anstelle des Kupfers der Schichten 1 und 2 entsprechende Mengen an Silberkristallen gleicher Korngröße verwendet werden.

Die Umsetzung und Aufarbeitung wird wie im Beispiel 1 beschrieben durchgeführt. Man erhält 386 Teile pro Stunde einer 32-gewichtsprozentigen Glyoxallösung, das sind 124 Teile Glyoxal pro Stunde,

5

entsprechend einer Ausbeute von 60,3 % der Theorie, bezogen auf verwendetes Glykol. Die Glyoxallösung hat einen Gehalt von 1,2 Gewichtsprozent Ethylenglykol, 0,9 Gewichtsprozent Formaldehyd, 6,1 Gewichtsprozent Glykolaldehyd und eine Säurezahl von 4. Der Umsatz beträgt 97,9 Prozent, die Raum-Zeit-Ausbeute 13,2 g Glyoxal pro cm$^3$ Katalysatorvolumen und Stunde. Farbzahl der Lösung nach dreitägigem Betrieb : 15.

## Beispiel 3

(Vergleichsbeispiel, bei dem der Schichtenkatalysator nur aus Kupfer besteht)

Es wird die gleiche Anlage wie im Beispiel 1 verwendet. In den Reaktor wird ein Schichtenkatalysator wie im Beispiel 1 eingetragen, wobei jedoch anstelle des Silbers der Schichten 3 und 4 entsprechende Mengen an Kupferkristallen gleicher Korngröße verwendet werden.

Die Umsetzung und Aufarbeitung wird wie im Beispiel 1 beschrieben durchgeführt. Man erhält 332 Teile pro Stunde einer 34-gewichtsprozentigen Glyoxallösung, das sind 113 Teile Glyoxal pro Stunde, entsprechend einer Ausbeute von 54,9 % der Theorie, bezogen auf verwendetes Glykol. Die Glyoxallösung hat einen Gehalt von 3,7 Gewichtsprozent Ethylenglykol, 0,9 Gewichtsprozent Formaldehyd, 1,2 Gewichtsprozent Glykolaldehyd und eine Säurezahl von 5. Der Umsatz beträgt 94,4 Prozent, die Raum-Zeit-Ausbeute 12,0 g Glyoxal pro cm$^3$ Katalysatorvolumen und Stunde. Farbzahl der Lösung nach dreitätigem Betrieb : 20.

## Beispiel 4

(Vergleichsbeispiel, bei dem als Katalysator eine Mischung von Kupfer sind Silber verwendet wird)

Es wird die gleiche Anlage wie in Beispiel 1 verwendet. In den Reaktor wird als Katalysator eine mechanische Mischung von Kupfer- und Silberkristallen mit der gleichen Korngrößen- und Gewichtsverteilung wie in Beispiel 1 angegeben eingefüllt. Die Gesamtmenge (41 Teile) ist die gleiche wie in Beispiel 1. Die Schütthöhe des Katalysators ist aufgrund des höheren Schüttgewichts der Mischung mit 27 mm etwas geringer.

Die Umsetzung und Aufarbeitung wird wie im Beispiel 1 beschrieben durchgeführt. Man erhält 362 Teile pro Stunde einer 33-gewichtsprozentigen Glyoxallösung, das sind 119 Teile Glyoxal pro Stunde, entsprechend einer Ausbeute von 57,8 % der Theorie, bezogen auf verwendetes Glykol. Die Glyoxallösung hat einen Gehalt von 1,9 Gewichtsprozent Ethylenglykol, 1,1 Gewichtsprozent Formaldehyd, 4,2 Gewichtsprozent Glykolaldehyd und eine Säurezahl von 4.

Der Umsatz beträgt 96,9 Prozent, die Raum-Zeit-Ausbeute 14,1 g Glyoxal pro cm$^3$ Katalysatorvolumen und Stunde. Farbzahl der Lösung nach dreitägigem Betrieb : 15.

## Beispiel 5

(Zusatz einer flüchtigen Phosphorverbindung)

Es wird die gleiche Anlage und der gleiche Mehrschichtenkatalysator wie in Beispiel 1 verwendet. Die Umsetzung wird bei 600 °C und 1,2 bar analog Beispiel 1 durchgeführt, wobei dem zum Katalysator strömenden Gasgemisch zusätzlich 0,003 Teile pro Stunde Trimethylphosphat, $(CH_3O)_3$ PO (entsprechend 3 ppm P, bezogen auf das Gewicht an zugeführtem Ethylenglykol) zugegeben werden.

Man erhält 353 Teile pro Stunde einer 45-gewichtsprozentigen Glyoxallösung, das sind 159 Teile Glyoxal pro Stunde, entsprechend einer Ausbeute von 77,3 % der Theorie, bezogen auf verwendetes Glykol. Die Lebensdauer des Katalysators beträgt 90 Trage. Die Glyoxallösung hat einen Gehalt von 0,05 Gewichtsprozent Ethylenglykol, 1,0 Gew.% Formaldehyd, 1,05 Gew.% Glykolaldehyd und eine Säurezahl von 5.

Der Umsatz beträgt 99,9 Prozent, die Raum-Zeit-Ausbeute 16,9 Glyoxal pro cm$^3$ Katalysatorvolumen und Stunde. Farbzahl der Lösung nach dreitätigem Betrieb : 11.

## Beispiel 6

(Vergleichsbeispiel mit einer größeren Menge einer flüchtigen Phosphorverbindung)

Es wird die gleiche Anlage wie in Beispiel 1 verwendet. In den Reaktor wird ein Mehrschichtenkatalysator aus Kupfer und Silber analog Beispiel 1 eingetragen. Die Umsetzung wird bei 600 °C und 1,2 bar analog Beispiel 5 durchgeführt, wobei aber hier 0,03 Teile pro Stunde Trimethylphosphat, $(CH_3O)_3$ PO (entsprechend 30 ppm P, bezogen auf das Gewicht an zugeführtem Ethylenglykol) dem zum Katalysator strömenden Gasgemisch zugeführt werden.

Man erhält 357 Teile einer 42-gewichtsprozentigen Glyoxallösung, das sind 150 Teile pro Stunde Glyoxal, entsprechend einer Ausbeute von 72,9 % der Theorie, bezogen auf verwendetes Glykol.

Die Glyoxallösung hat einen Gehalt von 1,1 Gewichtsprozent Ethylenglykol, 0,8 Gewichtsprozent Formaldehyd, 3,5 Gewichtsprozent Glykolaldehyd und eine Säurezahl von 3. Der Umsatz beträgt 98,2 Prozent, die Raum-Zeit-Ausbeute 16,0 g Glyoxal pro cm³ Katalysatorvolumen und Stunde. Farbzahl der Lösung nach dreitägigem Betrieb : 9.

In der Tabelle sind die wesentlichen Ergebnisse der erfindungsgemäßen Beispiele sowie der Vergleichsbeispiele zusammengestellt. Es wird deutlich, daß bei Verwendung des erfindungsgemäßen Kupfer-Silber-Schichtenkatalysators (1) die Glyoxalausbeute erheblich höher und der Gehalt der Glyoxallösung an störenden und schwer abtrennbaren Verunreinigungen deutlich niedriger ist als bei Verwendung eines reinen Silber- bzw. Kupferkatalysators (2 bzw. 3) oder einer Kupfer-Silber-Mischung (4). Der erfindungsgemäße Zusatz von 3 ppm Phosphor (5) bewirkt eine zusätzliche Ausbeuteerhöhung bei leicht verbesserter Produktqualität. Wird die Phosphormenge jedoch über den optimalen Wert hinaus gesteigert (6 mit 30 ppm Phosphor), so sinkt die Ausbeute und die Produktqualität verschlechtert sich.

| Beispiel | Umsatz Glykol (%) | Ausbeute Glyoxal (%) | Gehalt an Nebenprodukten ger. Glyoxal 40 % | | | Säurezahl 1) | Farbzahl 2) |
|---|---|---|---|---|---|---|---|
| | | | Glykol (Gew.%) | Glykolald. (Gew.%) | Formald. (Gew.%) | | |
| 1 (erfindungs-gemäß) | 99,9 | 70,6 | 0,05 | 0,9 | 1,2 | 4 | 12 |
| 2 (Vergleich) | 97,9 | 60,3 | 1,5 | 7,6 | 1,1 | 5 | 19 |
| 3 (Vergleich) | 94,4 | 54,9 | 4,4 | 1,4 | 1,1 | 6 | 24 |
| 4 (Vergleich) | 96,9 | 57,8 | 2,3 | 5,1 | 1,3 | 5 | 18 |
| 5 (erfindungs-gemäß) | 99,9 | 77,3 | 0,04 | 0,9 | 0,9 | 4 | 10 |
| 6 (Vergleich) | 98,2 | 72,9 | 1,0 | 3,3 | 0,8 | 3 | 9 |

1) Angabe der Kaliumhydroxidmenge in Milligram, die zur Neutralisation von einem Gramm Glyoxallösung benötigt wird

2) Bestimmung mittels Platin-Cobalt-Skala nach ASTM D 1209-69

**Patentansprüche**

1. Verfahren zur kontinuierlichen Herstellung von Glyoxal durch Oxidation von Ethylenglykol, bei dem man das Ethylenglykol mit Sauerstoff und einem Inertgas bei Temperaturen von 450 bis 800 °C über einen Katalysator aus Kupfer und Silber leitet, dadurch gekennzeichnet, daß der Katalysator aus einer oder mehreren Schichten von Kupferkristallen und einer oder mehreren Schichten von Silberkristallen besteht, wobei die Kupfer- und Silberkristalle eine Korngröße von 0,1 bis 2,5 Millimetern aufweisen und der Anteil an Silberkristallen 55 bis 90 Gew.%, jeweils bezogen auf das Gesamtgewicht des Katalysators, beträgt.

2. Verfahren nach Anspruch 1, dadurch gekennzeichnet, daß man die Oxidation in Gegenwart einer unter den Reaktionsbedingungen flüchtigen Phosphorverbindung durchführt, wobei die Phosphormenge (berechnet P), bezogen auf die Gewichtsmenge an Ethylenglykol, 0,5 bis 20 ppm beträgt.

3. Verfahren nach Anspruch 1, dadurch gekennzeichnet, daß der Katalysator aus ein bis vier Schichten von Kupferkristallen und ein bis vier Schichten von Silberkristallen besteht.

4. Verfahren nach Anspruch 1, dadurch gekennzeichnet, daß der Katalysator aus zwei oder drei Schichten von Kupferkristallen und zwei bis drei Schichten von Silberkristallen besteht.

# 0 072 912

**Claims**

1. A process for the continuous preparation of glyoxal by oxidation of ethylene glycol, in which the ethylene glycol, oxygen and an inert gas are passed at from 450 to 800 °C over a catalyst consisting of copper and silver, wherein the catalyst consists of one or more layers of copper crystals and one or more layers of silver crystals, the copper and silver crystals having a particle size of from 0.1 to 2.5 mm, and the amount of silver crystals being from 55 to 90 % by weight, based on the total weight of the catalyst.

2. A process as claimed in claim 1, wherein the oxidation is carried out in the presence of a phosphorus compound which is volatile under the reaction conditions, the amount of phosphorus (calculated as P) being from 0.5 to 20 ppm, based on the amount by weight of ethylene glycol.

3. A process as claimed in claim 1, wherein the catalyst consists of from one to four layers of copper crystals and from one to four layers of silver crystals.

4. A process as claimed in claim 1, wherein the catalyst consists of two or three layers of copper crystals and two or three layers of silver crystals.

**Revendications**

1. Procédé pour la préparation en continu de glyoxal par oxydation d'éthylène-glycol, en faisant passer l'éthylène-glycol avec de l'oxygène et un gaz inerte, à des températures de 450 à 800 °C, sur un catalyseur de cuivre et d'argent, caractérisé en ce que le catalyseur se compose d'une ou de plusieurs couches de cristaux de cuivre et d'une ou de plusieurs couches de cristaux d'argent, les cristaux de cuivre et d'argent présentant une grosseur de grain de 0,1 à 2,5 mm et la proportion de cristaux d'argent étant comprise entre 55 et 90 % en poids, par rapport au poids total du catalyseur.

2. Procédé selon la revendication 1, caractérisé en ce qu'on effectue l'oxydation en présence d'un composé du phosphore volatil dans les conditions de la réaction, la quantité de phosphore (P calculé) étant comprise entre 0,5 et 20 ppm par rapport à la quantité en poids d'éthylène-glycol.

3. Procédé selon la revendication 1, caractérisé en ce que le catalyseur se compose d'une à quatre couches de cristaux de cuivre et d'une à quatre couches de cristaux d'argent.

4. Procédé selon la revendication 1, caractérisé en ce que le catalyseur se compose de deux ou trois couches de cristaux de cuivre et de deux ou trois couches de cristaux d'argent.